Europäisches Patentamt

European Patent Office

Office européen des brevets

(19)

(11) Publication number: 0 381 331
A2

# EUROPEAN PATENT APPLICATION

(21) Application number: 90300460.4

(22) Date of filing: 17.01.90

(51) Int. Cl.5: C12N 15/58, C12P 21/02,
A61K 37/54

Claims for the following Contracting States: ES + GR

(30) Priority: 17.01.89 US 298157

(43) Date of publication of application:
08.08.90 Bulletin 90/32

(84) Designated Contracting States:
AT BE CH DE DK ES FR GB GR IT LI LU NL SE

(71) Applicant: INTEGRATED GENETICS, INC.
One Mountain Road
Framingham, MA 01701(US)

(72) Inventor: Livingston, David J.
32 Chandler Place
Newton, Massachusetts 02164(US)
Inventor: Manavalan, Parthasarathy
53 Auburn Street
Framingham, Massachusetts 01701(US)

(74) Representative: Sheard, Andrew Gregory et al
Kilburn & Strode 30, John Street
London WC1N 2DD(GB)

(54) Gene sequences encoding modified residues situated in the protease domain of tpa.

(57) There are provided methods for generating novel, modified DNA sequences for encoding modified polypeptides having tissue plasminogen activator (tPA) fibrinolytic-like activity. Preferred embodiments comprise alterations in the protease domain in order to more closely mimic the structures and activity of the urokinase protease domain.

Fig 1

# NOVEL GENE SEQUENCES ENCODING MODIFIED RESIDUES SITUATED IN THE PROTEASE DOMAIN OF TPA

This invention relates to the use of recombinant DNA techniques to produce therapeutic proteins with improved specific activities for plasminogen activation, in particular to the use of such techniques to produce novel, modified human tissue plasminogen activator (mtPA) genes and plasmids containing such genes, host cells transformed or transfected thereby, and mtPA molecules produced therefrom.

Tissue plasminogen activator (tPA) is a multi-domain serine protease which catalyzes conversion of plasminogen to plasmin As such, tPA is of therapeutic value. When administered exogenously, tPA can effect a lysis of blood clots (thrombolysis). tPA has been proven effective in clinical trials for treatment of myocardial infarction. Other indications being examined include pulmonary embolism, deep vein thrombosis and stroke.

tPA secreted by human melanoma cells was purified and characterized by Rijken et al. (J. Biol. Chem. 256, 7035 (1981). Therapeutic utility of exogenous tPA was demonstrated with the melanoma-derived material (US Patent No. 4,752,603, Collen et al., J. Clin. Inv. 71, 368 (1983); Korninger et al., J. Clin Inv. 69, 573 (1982)). Differences between tPA derived from melanoma and normal uterine tissue have been reported (Pohl et al., FEBS Lett. 168, 29 (1984)). Rijken et al., Biochem. Biophys. Acta 580, 140 (1979) describe the partial purification, from human uterine tissue, of human tissue plasminogen activator (utPA).

Recombinant DNA techniques have been used previously to obtain mRNA from a line of cancer cells (Bowes melanoma cells), this mRNA being used to produce cDNA encoding Bowes tPA, as described in Goeddel et al., US Patent No. 4,766,075. Copending, commonly assigned U.S.S.N. 782,686 to Wei et al., fully incorporated herein by reference, describes DNA sequences encoding uterine tPA. This reference further describes site-directed mutagenesis of the DNA sequence at any one or more of the three positions which code for amino acids which in turn normally become glycosylated in post-translation processing steps by mammalian cells. The resultant modified tPA molecules having altered amino acid sequences fail to exhibit glycosylation at the mutagenized site. The work has also been reported by Wei et al., DNA 4, 76 (1985), by Lau et al., Bio/Technology 5, 953 (1987) and in EPA 178,105. Expression vectors utilized in the present Invention for expression of secreted tPA and mtPA's in mouse C127 cells have been described by Reddy et al., J. Cell Biochem. 10D, 154 (1986).

The tPA molecule contains five discrete structural domains. In the presence of plasmin, single-chain tPA or zymogen enzyme can be cleaved into an activated two-chain form. The heavy chain contains four of these domains: a "finger" domain which is homologous to a portion of fibronectin; a "growth factor" domain which is homologous to epidermal growth factor; and two non- equivalent "Kringle" domains. Plasmin cleavage to form two-chain tPA occurs C-terminal to Kringle 2 (at Arg278). The light chain contains the serine protease domain, which is homologous to trypsin and chymotrypsin.

tPA is a relatively clot-specific plasminogen activator due to its affinity for fibrin, which forms the clot matrix. This fibrin affinity is believed to be due to interactions of the finger and Kringle 2 domain with fibrin. For reasons poorly understood, Kringle 1 interacts more weakly than Kringle 2 with fibrin (van Zonneveld et al., Proc. Natl. Acad. Sci. USA 83: 4670 (1986)). The different affinities might be due, for example, to different orientations of the domains relative to the serine protease domain.

A large number of serine proteases have been sequenced and many have been crystallized. Study of the crystallized enzymes by X-ray diffraction techniques has led to determination of the three dimensional structures of the serine proteases trypsin, chymotrypsin, subtilisn, elastase and kallikrein. It is thought that examination of these structures and experimental data on specificity for the substrate can give insight into how enzyme active site structure and charge determines specificity and it is one aspect of the present invention to utilize such data to derive new methods of designing molecules.

Urokinase (UK) and tPA both catalyze the same reaction - cleavage of the Arg-Val bond of plasminogen. These enzymes perform this reaction with very different catalytic efficiency. The second order rate constants for plasminogen activation by urokinase (UK) or tPA are 38 and 4.8 $\mu M^{-1}$ $min^{-1}$, respectively - an eight-fold higher efficiency for UK (Lee et al., Biochemistry 27, 7506 (1988)). However, in contrast to UK, tPA has the desireable property of fibrin-enhancement of its catalytic activity. Estimates of the degree of fibrin stimulation have exceeded 2000-fold (de Vries et al., Biochemistry 27, 2565 (1988)).

It is an aspect of the present invention to provide agent an improved thrombolytic agent possessing the high catalytic efficiency of UK and the fibrin-specificity of tPA.

Previous attempts to develop such an improved agent led investigators to generate tPA-UK hybrids by site-directed mutagenesis (de Vries et al., ibid). In these attempts, the fibrin-binding regions of tPA were fused to the serine protease domain of UK. While such hybrids cleave plasminogen at higher rates than tPA in the absence of fibrin, they have lost the desired degree of fibrin-dependence shown by the parent tPA protein.

It is another aspect of the present invention to provide novel molecules possessing the desired properties and overcoming the deficiencies of the conventional chimera approach.

It is yet another aspect of the present invention to provide novel methods for predictably insuring the tPA cDNAs are altered in the desired manner to produce the desired modified tissue plasminogen activator molecules (mtPAs).

In accordance with the various principles and aspects of the present invention, there is provided a novel approach to the improvement of the catalytic efficiency of tPA comprising aligning the tPA serine protease domain sequence with other serine protease sequences (most ideally UK), molecular modeling of the predicted tertiary structure of tPA, and identifying mutations in the tPA molecule which increase the molecule's catalytic efficiency. Accordingly, there also are provided mtPA's which are generated, using this approach, from the parent tPA molecule by substitution of selected amino acids in the protease domain. It has also been surprisingly discovered that differences between the primary sequences of the protease regions of tPA and UK are responsible for their different catalytic effiencies in the absence of fibrin. Without wishing to be constrained by theory, it is hypothesized that certain amino acid residues in the plasminogen-binding region of the tPA protease domain provide favorable binding energies for portions of the plasminogen molecule and that these interactions can be increased by substitution of different amino acids at these postions. The procedure and molecules of the present invention provide novel approaches for generating new molecules having a biological activity associated with tissue plaminogen activator which takes into account these unexpected findings. It was found additionally useful on this regard to employ structures of the active site regions of other serine proteases to predict the three-dimensional structure of the protease domain of tPA. It was surprising to discover that such alterations in the protease domain still result in molecules having a biological activity higher than wild-type or unmodified tPA since it was more likely that such mutations might have caused incorrect folding, defective secretion or decreased activity of the mtPA.

A preferred method of this invention for the production of desired modified tPA molecules comprises the alteration of residues in tPA to more closely mimic comparable residues in UK. The most preferred nucleotide and resultant amino acid substitutions in the protease domain (indicated by underlining) comprise those sequences listed in Table 4. Those skilled in the art will recognize that two or more of these sequences can be combined or altered by additional amino acid replacements or insertions and that all such modifications are included provided they do not substantially lower the catalytic activity of the protein below that possessed by tPA.

Another preferred method of this invention comprises introducing at least one altered amino acid into the tPA protein sequence by altering the cDNA encoding the tPA. (While cDNA has been specified and described throughout this specification, such is simply to facilitate discussion and understanding and is not to be construed as limiting; those skilled will readily recognize that genome encoding tPA can be used in substitution of cDNA in carrying out the novel inventions described herein.) In one embodiment, these alterations are made by introducing or removing unique restriction sites so that the presence of these mutations can be confirmed by restriction digest. In the most preferred embodiments, these modifications have been combined with other modifications for the purpose of obtaining molecules with extended in vivo half-life, for example the conversion of an asparagine to a glutamine at residue 120 or at residue 451, such a conversion being represented herein as N120Q, or N451Q, respectively (wherein N stands for asparagine and Q stands for glutamine pursuant to single letter-amino acid convention set forth hereafter). Another preferred embodiment comprises the combination of the mutations of the present invention with other deletions of the finger or EGF-homologous domains of tPA. Such other deletion mutants have been reported to extend half-life (Collen et al., Blood 71, 216 (1988)).

Also provided by the instant invention are transformed host organisms for maintenance and replication of the sequences and for production of the preferred mutant molecules; expression vectors for expression of the modified sequences encoding the mutant or modified tPA (mtPA) molecules in COS, C127, and CHO cells; and the mtpA proteins derived from these expression systems.

Further understanding of the instant invention may be had by reference to the drawings wherein:

Figure 1 - depicts expression vector LK444BHS for expressing the novel mtPA's in COS cells;

Figure 2 - depicts expression vector CLH3AXSV2DHFR for expressing the novel mtPA's in CHO cells; and

Figure 3 - depicts expression vector CLH3AXBPV for expressing the novel mtPA's in C127 cells.

The term "tissue plasminogen activator" or "tPA" refers to the protein derived from natural sources or produced by recombinant DNA techniques in microbial cells or cell culture. It is capable of binding to fibrin and of catalyzing the conversion of plasminogen to plasmin.

The term "modified tissue plasminogen activator" or "mtPA" refers to a variant of tPA which has been altered by removal, insertion, duplication or replacement of amino acids. These may be naturally-occurring alterations due to allelic variation or may be artificial alterations generated by site-directed mutagenesis of the cDNA or gene encoding the protein.

The term "cell culture" refers to the containment of growing cells typically derived from either a multicellular plant or animal which allows for the cells to remain viable outside the original plant or animal.

The term "host cell" refers to a microorgansim including yeast, bacteria and eukaryotic cells including, without limitation, mammalian cells which can be grown in cell culture and transfected or transformed with a plasmid or vector containing a gene encoding a molecule having a tPA biological characteristic for expression of such molecule.

The term "domain" refers to a discrete continuous part of an amino acid sequence that can be equipped with a particular function. With respect to tPA, useful reference in this regard include those of Banyai, L. et al., Common evolutionary origin of the fibrin-binding structures of fibronectin and tissue-type plasminogen activator, FEBS Lett. 163(1), 37-41 (1983) and Ny, T. et al., The structure of the Human Tissue-type Plasminogen Activator Gene: Correlation of Intron and Exon Structures to Functional and Structural Domains, Proc. Natl. Acad. Sci. USA 81, 5355-5359 (1984) which have defined the domain regions. Tables 2 and 3 herein show the approximate, generally accepted locations of the domain regions.

The term "downstream" identifies sequences proceeding farther in the direction of expression; for example, the coding region is downstream from the initiation codon.

The term "interdomain" refers to the regions of a protein's amino acid sequence that lie between the domains.

The term "maintained" refers to the stable presence of a plasmid within a transformed host wherein the plasmid is present as an autonomously replicating body or as an integrated portion of the host's genome.

The term "microorganism" includes both single cellular prokaryote and eukaryote organisms.

The phrase "non-native endonuclease restriction sites" refers to endonuclease restriction sites that are not normally present in the native cDNA and are engineered by site-directed mutagenesis into the cDNA nucleotide sequence.

The term "operon" is a complete unit of gene expression and regulation, including structural genes, regulator genes, and control elements in DNA recognized by regulator gene product.

The term "plasmid" refers to an autonomous self-replicating extrachromosomal circular DNA and includes both the expression and nonexpression types. Where a recombinant microorganism or cell culture, which provides expression of such molecule, is described as hosting an expression plasmid, the term "expression plasmid" includes both extrachromosomal circular DNA and DNA that has been incorporated into the host chromosome(s).

The term "promoter" is a region of DNA involved in binding the RNA polymerase to initiate transcription.

The term "DNA sequence" refers to a single- or double-stranded DNA molecule comprised of nucleotide bases, adenosine, thymidine, cytosine and guanosine and further includes genomic and copy DNA (cDNA).

The term "suitable host" refers to a cell culture or microorganism that is compatible with a recombinant plasmid and will permit the plasmid to replicate, to be incorporated into its genome or to be expressed.

The term "upstream" identifies sequences proceeding in the opposite direction from expression. "Upstream" also identifies nucleotides which precede other nucleotides in the 5′ direction. For example, the bacterial promoter is upstream from the transcription unit, the initiation codon is upstream from the termination codon.

The term "restriction endonuclease" alternatively referred to as restriction enzymes refers to enzymes which cleave double-stranded DNA (dsDNA) at locations or sites characteristics to the particular enzyme. For example, the restriction endonuclease EcoR1 cleaves dsDNA only at locations:

```
5'GAATTC3'    to form    5'G        and    AATTC3'    fragments.
3'CTTAAG5'               3'CTTAA            G5'
```

Although many of such enzymes are known, the most preferred embodiments of the present invention are primarily concerned with only selected restriction enzymes having specified characteristics.

Conventions used to represent plasmids and fragments are meant to be synonymous with conventional representations of plasmids and their fragments. Unlike the conventional circular figures, the single line figures on the charts or tables represent both circular and linear double-stranded DNA with initiation or transcription occurring from left to right (5$'$ to 3$'$). Numbering of nucleotides and amino acids correspond to the particular amino ($NH_2$) terminal form shown in Table 2, although it will be readily understood that obvious numbering modifications may apply with different $NH_2$ terminal forms. The table below provides the standard abbreviations for amino acids.

| Abbreviations for amino acids | | |
|---|---|---|
| Amino Acid | Three-letter abbreviation | One-letter symbol |
| Alanine | Ala | A |
| Arginine | Arg | R |
| Asparagine | Asn | N |
| Aspartic acid | Asp | D |
| Cysteine | Cys | C |
| Glutamine | Gln | Q |
| Glutamic acid | Glu | E |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

In relation to the earlier mentioned discovery that differences between the primary sequences of the serine proteases are responsible for differences in catalytic efficiency, comparisons were made between the primary sequences of trypsin, chymotrypsin, elastase, kallikrein, UK and tPA, the latter two being shown in Table 1. Computer-generated images of the protease regions of tPA and UK were made based on published X-ray coordinates of four serine proteases. Comparison of the catalytic triads and active-site binding pockets of tPA and UK showed a number of amino acid differences believed to be associated with the increased affinity of the tPA active site for binding to the tripeptide Pro-Gly-Arg sequence found at the cleavage site of plasminogen. The present invention provides novel methods for advantageously manipulating these newly discovered differences.

## General Methods

Methods of DNA preparation, restriction enzyme cleavage, restriction enzyme analysis, gel electrophoresis, DNA fragment isolation, DNA precipitation, DNA fragment ligation, bacterial transformation, bacterial colony selection and growth are conventional procedures in this art and are extensively detailed in Maniatis et al., Molecular Cloning: A Laboratory Manual, Cold Spring Harbor, New York 1982 (hereafter referred to as Maniatis). Methods of in vitro RNA transcription in a buffered medium and in vitro protein translation in rabbit reticulocyte lysate are as detailed in the manufacturer's instructions (Promega Biotech). DNA sequencing was performed via the Sanger dideoxy method using either single-stranded DNA or denatured double-stranded DNA.

## tPA cDNA Source

The cloning of the full-length cDNA of human uterine tPA is described by Reddy et al. (1987). Essentially, mRNA was made from human uterine tissue by the guanidine thiocyanate procedure followed by CsC1 gradient purification and oligo-dT affinity purification. Reverse transcriptase and Klenow were used to convert the message into double-stranded cDNA which was then cloned into the PstI site of pBR322. The tPA cDNA clone was screened for with oligonucleotides deduced from the sequence of Bowes melanoma tPA. A 2455 base pair cDNA was isolated, sequenced and found to be in good agreement with published sequences (Pennica et al., 1983). The uterine tPA cDNA differed from melanoma tPA at several sites (predominantly in the 3̄ untranslated region of the clone) (from Reddy et al., 1987).

An Sfan1 site (nucleotide 16) at the 5′ end of the clone near the A̅T̅G̅ start codon for tPA and BglIII site (2090) was cleaved, filled in with Klenow in the presence of dNTP's, and SalI linkers lygated to the blunt ends. The cDNA was recloned into pBR322 as a SalI fragment and subsequently recloned into other vectors using the SalI sites.

Synthesis of Primers for Site-Specific Mutagenesis

The human uterine tPA cDNA was modified by site-specific mutagenesis using synthetic oligonucleotides prepared by the solid phase phosphotriester method.

The following primers were synthesized and used for such mutagenesis. They are in the anti-sense sequence.

1. Primer PP1 (21-mer)
5′ GCC.C̅C̅C̅.G̅C̅A̅.A̅A̅C̅.GAA.CCG.CTC 3′
2. Primer PP2 (21-mer)
5′ CGG.A̅A̅A̅.C̅C̅G̅.ATC.CTG.GAA.GCA 3′
3. Primer PP3 (21-mer)
5′ AGT.G̅T̅C̅.G̅G̅C̅.ATC.GAA.CTC.CTT 3′
4. Primer PP4 (21-mer)
5′ GTC.A̅T̅T̅.G̅T̅C̅.GAG.CGT.GTC.ATC 3′
5. Primer PP5E (21-mer)
5′ GTA.C̅A̅G̅.T̅C̅T̅.CTC.ATG.AGC.CTC 3′
6. Primer PP5Q (21-mer)
5′ GTA.C̅A̅G̅.T̅C̅T̅.CTG.ATG.AGC.CTC 3′
7. Primer PP6S (21-mer)
5′ CCG.A̅G̅T̅.G̅T̅C̅.GCT.AGC.ACA.CAG 3′
8. Primer PP6T (21-mer)
5′ CCG.A̅G̅T̅.G̅T̅C̅.TGT.AGC.ACA.CAG 3′
9. Primer PP6D (21-mer)
5′ CCG.A̅G̅T̅.G̅T̅C̅.GTC.AGC.ACA.CAG 3′
10. Primer PP7 (27-mer)
5′ CTG.G̅G̅G̅.G̅C̅C̅.G̅C̅C̅.GGG.CAG.AGT.GTC.TCC 3′
11. Primer PP8 (21-mer)
5′ GCC.C̅T̅G̅.G̅C̅A̅.G̅C̅T̅.GTC.GTG.CAA 3′
12. Primer PP9 (21-mer)
5′ GCC.C̅C̅A̅.G̅C̅T̅.C̅A̅C̅.GAT.GCC.CAC 3′
13. Primer PP10A (21-mer)
5′ ATC.C̅T̅T̅.C̅T̅G̅.AGC.ACA.GCC.CAG 3′
14. Primer PP10S (21-mer)
5′ ATC.C̅T̅T̅.C̅T̅G̅.A̅C̅T̅.ACA.GCC.CAG 3′
15. Primer PP11D (21-mer)
5′ GTA.C̅A̅C̅.A̅C̅C̅.G̅T̅C̅.GAC.ATC.CTT 3′
16. Primer PP3 (21-mer)
5′ GTA.C̅A̅C̅.A̅C̅C̅.C̅T̅G̅.CAC.ATC.CTT 3′

Site Specific Mutagenesis using above Synthesized Oligonucleotides

The M13 based oligonucleotide directed mutagenesis procedure employed was essentially as detailed by Kunkel et al. (Proc. Nat'l. Acad. Sci. USA 82, 488 (1985)). Phage containing the m13tPA.MGB vector (the tPA cDNA which has the Asn 120 Gln mutation described in the aforementioned copending application and publication of Wei et al.) were grown in an E.coli strain CJ236 (which is dut⁻ ung⁻) in media in the presence of 0.5 ug/ml uridine. The single stranded DNA produced contained uracil residues instead of thymine. The single stranded M13mp18.mtPA was prepared by conventional procedures.

10 ng of the phosphorylated mutating oligonucleotide was annealed with 1 ug of single stranded DNA in a total volume of 20 ul containing 1 x SSC (0.15 M NaCl, 15 mM sodium citrate). The annealing mixture was heated to 70° C then allowed to cool slowly in a water bath for several hours. The annealed fragments were converted to covalently closed circular DNA by the action of DNA polymerase and T4 ligase. The annealed mixture was made up to 100 ul containing 20 mM Hepes (pH 7.8), 2 mM DTT, 10 mM MgCl₂, 500 uM each of dATP, dTTP, dCTP, and dGTP, 1mM ATP, 2.5 units of Klenow and 2 units of T4 DNA ligase. Incubation started at 0° C for 5 minutes and proceeded at room temperature for 5 minutes, 37° C for 2 hours, and then overnight at 16° C.

10 ul of the above reactive mixture was added to 300 ul of competent E. coli strain DH₅ (American Type Culture Collection (ATCC)) bacterial cells and left in ice for 1 hour. The bacteria were heat shocked at 37° C for 5 minutes then serially diluted into 3 ml of soft agar (0.6%) containing 200 ul of mid-log phase E. coli strain JM101 (available from the ATCC) and poured onto LB agar plates and allowed to solidify. The plates were then incubated overnight at 37° C.

Nitrocellulose lifts were taken from the plates, baked at 80° C, under vacuum, for 2 hours and hybridized with the desired ³²P- labeled mutant oligonucleotide selected from the group of primers described above. Mutant positive isolates were determined by thermal denaturation of the DNA:oligo duplexes and further grown in JM101 on a larger scale for maxi-prep Rf DNA isolation. The BamHI-HindIII fragment containing the tPA cDNA including the Asn 120 to Gln mutation and the new mutation was further recloned into the SP65 vector and/or mammalian cell expression vector using normal procedures of restriction enzyme cleavage and ligation.

An example of this method is the generation of mutant V436E wherein the first letter indicates the original amino acid, the number is the sequence site number, and the second letter indicates the new amino acid at that site:

433                                      439

Glu Ala His Val Arg Leu Tyr

GAG GCT CAT GTC AGA CTG TAC

1405                      -     1425

Site-specific, oligonucleotide-directed mutagenesis using the PP5E primer (5′ GTA CAG TCT CTG ATG AGC CTC 3′) yielded

433                                        439

Glu Ala His <u>Glu</u> Arg Leu Tyr

GAG GCT CAT <u>GAG</u> AGA CTG TAC

1405                                   1425

The above mutation was designed such that a diagnostic restriction enzyme site was introduced. In the case of PP5E, a BspHI recognition site (TCATGA) was engineered into the mutant cDNA region. This allowed for convenient screening for mutants and is further useful for monitoring for the presence of a mutation when various mutantations are combined. Vectors encoding other recombinant modified tPA molecules having the altered protease regions listed in Table 4 are similarly constructed with the primers identified in Table 4.

## Verification of mtPA cDNA structure

Mutations L309V; D368A; RS465,466LP; and A476S have been verified by conventional restriction enzyme analysis, sequencing and/or in vitro transcription/translation analysis. Other described molecules identified by application of the instant inventive methods are in process. Mutant encoded proteins are ideally also analyzed by zymography and compared to wild type tPA zymography studies to confirm desired fibrinolytic activity occurring via a molecule having the correct molecular weight.

## Vectors and Transfections

### Sp65-mtPA

The BamHI-HindIII fragment containing the tPA cDNA sequence is then ideally isolated from the above described M13mp18.mtPA by restriction enzyme cleavage and gel electrophoresis and ligated into the BamHI-HindIII cleaved SP65 vector (available commercially from Promega Biotech). This orientation (with the 5' end of the cDNA adjacent to the SP6 promoter) enables an analysis of the mutant protein product by in vitro RNA synthesis and in vitro protein synthesis. The SP65.mtPA vector is also a convenient vector to use during the manipulation of the inserted cDNA.

### LK444BHS.mtPA

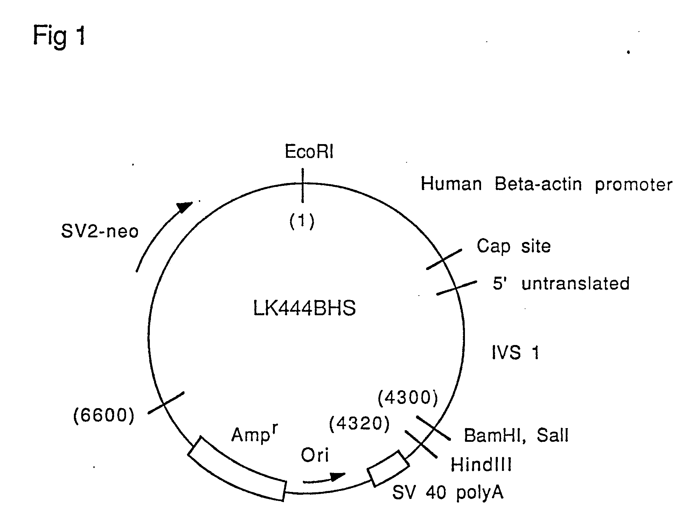

The vector shown in Figure 1 was constructed as follows. The bacterial vector region containing the ampicillin resistance gene, the origin of replication and the SV40 late polyadenylation signal sequence was derived from plasmid pCDV1 (Okayama and Berg, (1983) Mol. Cell. Biol. 3: 280). These sequences comprise nucleotides 4320 to 6600 of Figure 1. The cloning region polylinker (nucleotides 4300 to 4320) was derived from the SP64 polylinker (Melton et al.(1984) Nucl. Acids Res. 12: 7035). The promotor, cap site, 5' untranslated region and the first intron were from the gene encoding human beta actin (nucleotide numbers 1 to 4300 of Figure 1). The gene is described in Ng et al., Mol. Cell. Biol. 5: 2720 (1985). The neomycin transcriptional unit (nucleotides 6600 to 10,000) contains the bacterial neomycin resistance gene linked to the SV40 origin and early promotor (Southern and Berg, J. Mol. Appl. Genet. 1: 327 (1982)).

Referring to Figure 1, mutated cDNA molecules are advantageously recloned into the LK444BHS vector as follows. The BamHI-HindIII fragment containing the cDNA encoding the new mtPA is isolated from SP65.mtPA by restriction enzyme cleavage and gel filtration. This fragment is then ligated to a BamHI-HindIII cleaved vector LK444BHS. This allows for the transient expression of the tPA analogue in a COS 7 cell line driven by the human β-actin promoter.

### Transfection of COS cells

A transient expression system is advantageously used wherein the expression vector (LK444BHS.mtPA) is used to transfect COS-7 cells deposited at the ATCC as #CRL1651. Two to three days after introduction of the foreign DNA, conditioned medium is analyzed to characterize the activity of the secreted modified tPA protein. This procedure is carried out as follows.

$3 \times 10^6$ cells are grown in 100 mm plates in DMEM + 10% glutamine for 1 day preceding transfection. Ten to 20 mg of DNA is added to 2.0 ml of Tris-buffered saline (pH 7.5) and 1 ml of 2 mg/ml DEAE-dextran (made just before transfection by adding 50 mg DEAE-dextran + 25 ml TBS) is added to this solution. Cells are washed 2 times with phosphate-buffered saline (PBS) and the transfection solution added. Cells are incubated at 37°C for 15 -30 minutes, Dextran solution removed, and cells washed again with PBS 2 times. This solution is then replaced with 10 ml DMEM medium (no serum) plus 100 ml chloroquine (10 mM). The cells are then incubated at 37°C for 4 hours, washed twice with PBS, and fed with GIT serum free medium (10 ml).

### CLH3AXSV2DHFR

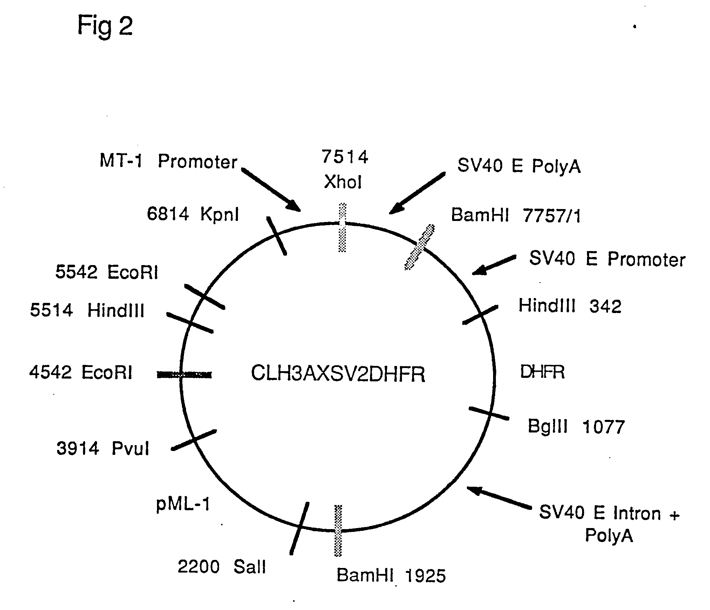

The vector, shown in Figure 2, was constructed from the dihydrofolate reductase transcriptional unit, which comprises the SV40 early region promotor, the mouse DHFR gene, and the SV40 small T intron and early region polyadenylation signal sequences (Reddy et al., DNA 6: 461 (1978)). These sequences comprise nucleotides 1 to 1925 of Figure 2. The bacterial plasmid sequences of the pML vector (Lusky and Botcham, Nature 283: 79 (1982)) provided nucleotides 2201 to 4542. The sequences encode the ampicillin resistance gene and the origin of bacterial replication. The metallothionein promotor (nucleotides 4542 to 7514 of the vector) was derived from the mouse metallothionein-1 gene with the introns and polyA sequences removed (Glanville et al., Nature 292: 267 (1981); Hamer and wailing, J. Mol. Appl. Genet. 1: 273 (1982)).

The SV40 early region polyadenylation signal sequences are from the vector described by Reddy et al, 1978. These sequences form nucleotides 7514 to 7751 of Figure 2.

The M13mp18.mtPA tPA cDNAs can be restriction enzyme digested with SalI and recloned into the vector cleaved with XhoI. The orientation of the tPA cDNA or mutant is such that expression is driven by the metallothionein promoter. The CLH3AXSV2DHFR.mtPA vector is used for the stable expression of the mtPA in CHO cells with the ability for methotrexate amplification.

Transfection of DHFR-CHO Cells

DUKX CHO cells were obtained from Lawrence Chasin of Columbia University (New York). These cells are deficient in dihydrofolate reductase. This gene is present in vector CLH3AXSV2DHFR. Cells can be plated in alpha plus media 10% FBS, 1% glutamine medium at a density of $7 \times 10^5$ cells per 100 mm dish 24 hours before transfection. 100-50 mg of plasmid DNA in 0.5 ml transfection buffer (the composition of which is 4 g NaCl, 0.185 g KCl, 0.05 g $Na_2HPO_4$, 0.5 g dextrose, and 2.5 g HEPES, pH 7.5 per 500 ml total volume). 30 ml of 2M $CaCl_2$ is added to the above solution and the mixture allowed to equilibrate for 45 minutes at room temperature. The medium is removed from the dishes, cells washed twice with PBS, and the DNA solution added to the cells. The cells are allowed to incubate at room temperature for 20 minutes. 5 ml of medium is then added and the cells incubated for four hours at 37° C. The media is then removed and the cells shocked with 15% glycerol in transfection buffer at 37° C for 3.5 minutes. After 48 hours, the cells are ideally split at a 1:3 ratio and fed with a selection medium containing 0.02 mM methotrexate. Cell colonies which survive the treatment appear 10 to 14 days after transfection.

Thereafter, selected colonies can be amplified with increasing levels of methotrexate according to published procedures (e.g. Michel et al., Bio/Technology 3, 561 (1985); U.S. 4,656,134 to Ringold). Modified tPA proteins produced by these cells can then be purified by previously reported procedures (Lau et al., Bio/Technology 5, 953 (1987).

CLH3AXBPV.mtPA

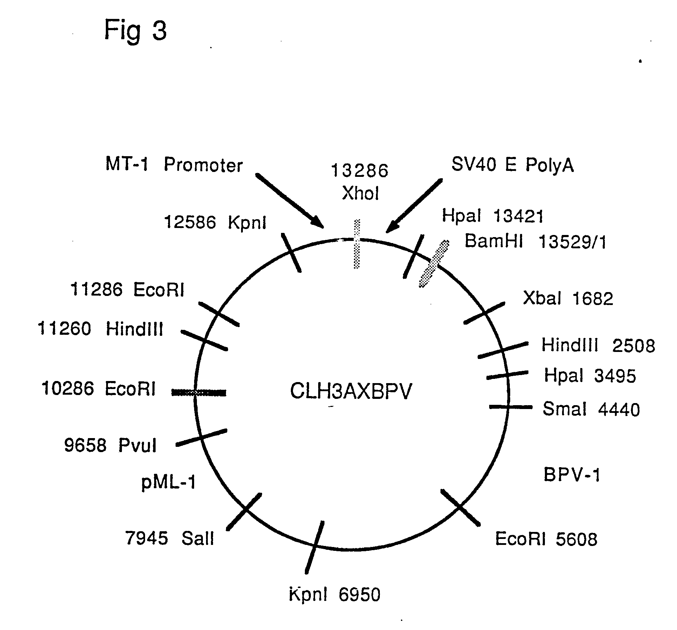

The vector shown in Figure 3 was constructed from the entire bovine papillomavirus (BPV) genome which was linearized as a BamHI fragment and one of the cohesive ends converted to a SalI site (Reddy et al., 1987). These sequences comprise nucleotides 1 to 7945 of the depicted vector. The bacterial plasmid sequences from pML encoding the ampicillin resistance gene and bacterial origin of replication (Lusky and Botcham, 1981) are at nucleotides 7945 to 10286. The metallothionein promotor was derived from the vector described by Glanville et al. (1981) and Hamer and Walling (1982). This sequence is found at nucleotides 10286 to 13286 of CLH3AXBPV. The SV40 early polyA sequences (Reddy et al., 1978) are found at nucleotides 13286 to 13529.

The SP65.mtPA cDNAs can be restriction enzyme digeste with SalI and the fragments isolated by gel purification. The SalI fragment can be ligated to an XhoI cleaved vector CLH3AXBPV to form CLH3AXBPV.mtPA. The orientation is advantageously determined and selected such that the inserted sequence is under the driving force of the metallothionine promoter in C127 cells.

Transfection of C127 Cells

Mouse C127 cells can then be transfected with the CLH3AXBPV.mtPA DNA preparations according to methods previously published by Hsiung et al., J. Mol. Appl. Genetics 2, 497 (1984).

Modified tPA proteins can then be purified from conditioned medium by previously reported procedures (Lau et al., Bio/Technology 5, 953 (1987)).

Assays of Modified tPA's

Quantitation of mtPA proteins in conditioned medium was performed with a commercially available ELISA Kit from American Diagnostica (Greenwich, CT, USA). The coating and detection antibody is a goat anti-human tPA IgG.

Activity is ideally determined by a published spectrophotometric assay for the rate of activation of plasminogen (Verheijen et al., Thromb. Haemostas. 48, 266 (1982)). The absorbance change measured in the assay is converted to Units by reference to a WHO melanoma tPA standard. Specific activity of the mtPA proteins can then be calculated by dividing Units by mgs of protein, the latter as determined in the ELISA assay.

Fibrin Affinity Assay

Alternatively, the protocol described by Nelles et al. (1987) J. Biol. Chem. 262, 10855 can be used to determine the affinity of the new mtPA's molecules for fibrin.

Pharmaceutical Applications

The mtPAs of the invention may advantageously be admixed with a pharmaceutically acceptable carrier substance, e.g., saline, and administered orally, intravenously, or by injection into affected arteries of the heart. Administration will be generally as is carried out for currently used blood clot lysing enzymes, approved tPA, streptokinase and urokinase.

The mtPA's of the invention may also be used therapeutically to lyse clots in human patients needing treatment of embolisms, e.g., post-operative patients, patients who have recently suffered myocardial infarction resulting in clots, and patients suffering from deep vein thrombi. The following examples are illustrative.

Example 1

For emergency treatment of thrombi by bolus injection, 5-10mg or other therapeutically effective amount of lyophilized mtPA are mixed together with saline and placed in the chamber of a syringe, which is used to inject the mtPA bolus into the patient intravenously.

Example 2

For infusion treatment for the rapid lysis of coronary thrombi, about 100mg/hr of lyophilized mtPA are infused intravenously over a period of about one hour, followed by intravenous infusion of about 50 mg/hr over a period of about three more hours.

Example 3

For infusion treatment for the rapid lysis of coronary thrombi, the protocol of Example 2 is followed, except that infusion is preceded by the intravenous injection of a bolus of about 10 mg mtPA in saline.

Example 4

For infusion treatment for the slow lysis of deep vein thrombi about mg/hr of lyophilized mtPA dissolved in saline are infused intravenously over a period of about 12-24 hours.

It will now be readily recognized by those skilled in the art that the foregoing amounts are merely representative and are subject to variation depending on the individual fibrinolytic characteristics of the particular mtPA selected. It will also be readily apparent that numerous modifications based on the teachings within may be made without departing from the spirit or scope of the present invention, and in particular but without limitation, the mtPAs of the present invention may be used for diagnostic purposes including in vitro assays and perhaps also for in vivo imaging applications and further, additional modifications may be made without departing from either the spirit or the scope of the application.

## TABLE 1

### Amino Acid Sequence Alignments of Tissue Plasminogen Activator (tPA) and Urokinase (UK) Protease Domains

```
                    280              290              300
    TPA   :    I K G G L F A D I A S H P W Q A A I F A K H R R S P
    UK    :    I - G G E F T T I E N Q P W F A A I Y R R H R G - -


            310              320              330
    G E R F L C G G I L I S S C W I L S A A H C F Q E R F P P H H
    S V T Y V C G G S L I S P C W V I S A T H C F I D Y P K K E D


            340              350              360
    L T V I L G R T Y R V V P G E E E Q K F E V E K Y I V H K E F
    Y I V Y L G R S R L N S N T Q G E M K F E V E N L I L H K D Y


            370              380              390
    D D D T Y D - - N D I A L L Q L K S D S S R C A Q E S S V
    S A D T L A H H N D I A L L K I R S K E G R C A Q P S R T


            400              410              420
    V R T V C L P P A D L Q L P D W T E C E L S G Y G K H E A L S
    I Q T I C L P S M Y N D P Q F G T S C E T T G F G K E N S T D


            430              440              450
    P F Y S E R L K E A H V R L Y P S S R C T S Q H L L N R
    Y L Y P E Q L K M T V V K L I S H R E C Q Q P H Y Y G S
```

```
            460                 470                 480
T V T D N M L C A G D T R S G G P Q A N L H D A C Q G D S G G
E V T T K M L C A A D P Q W K T - - - - - - D S C Q G D S G G


            490                 500                 510
P L V C L N D G R M T L V G I I S W G L G C G Q K D V P G
P L V C S L Q G R M T L T G I V S W G R G C A L K D K P G


            520                 530
V Y T K V T N Y L D W I R D N M R P
V Y T R V S H F L P W I R S H T K E
```

## TABLE 2

Nucleic Acid and Amino Acid Sequence for uterine pre-pro-tPA

```
    TGTGAAGCAATCATGGATGCAATGAAGAGAGGGCTCTGCTGTGTGCTGCTGCTGTGTGGA
  1 ---------+---------+---------+---------+---------+---------+ 60
    ACACTTCGTTAGTACCTACGTTACTTCTCTCCCGAGACGACACACGACGACGACACACCT

aa  C   E   A   I   M   D   A   M   K   R   G   L   C   C   V   L   L   L   C   G   -
```

```
    GCAGTCTTCGTTTCGCCCAGCCAGGAAATCCATGCCCGATTCAGAAGAGGAGCCAGATCT
 61 ---------+---------+---------+---------+---------+---------+ 120
    CGTCAGAAGCAAAGCGGGTCGGTCCTTTAGGTACGGGCTAAGTCTTCTCCTCGGTCTAGA

aa  A   V   F   V   S   P   S   Q   E   I   H   A   R   F   R   R   G   A   R   S   -
```

```
    TACCAAGTGATCTGCAGAGATGAAAAAACGCAGATGATATACCAGCAACATCAGTCATGG
121 ---------+---------+---------+---------+---------+---------+ 180
    ATGGTTCACTAGACGTCTCTACTTTTTTGCGTCTACTATATGGTCGTTGTAGTCAGTACC

aa  Y   Q   V   I   C   R   D   E   K   T   Q   M   I   Y   Q   Q   H   Q   S   W   -
```

```
    CTGCGCCCTGTGCTCAGAAGCAACCGGGTGGAATATTGCTGGTGCAACAGTGGCAGGGCA
181 ---------+---------+---------+---------+---------+---------+ 240
    GACGCGGGACACGAGTCTTCGTTGGCCCACCTTATAACGACCACGTTGTCACCGTCCCGT

aa  L   R   P   V   L   R   S   N   R   V   E   Y   C   W   C   N   S   G   R   A   -
```

```
         CAGTGCCACTCAGTGCCTGTCAAAAGTTGCAGCGAGCCAAGGTGTTTCAACGGGGGCACC
     241 ---------+---------+---------+---------+---------+---------+ 300
         GTCACGGTGAGTCACGGACAGTTTTCAACGTCGCTCGGTTCCACAAAGTTGCCCCCGTGG

     aa    Q  C  H  S  V  P  V  K  S  C  S  E  P  R  C  F  N  G  G  T  -


         TGCCAGCAGGCCCTGTACTTCTCAGATTTCGTGTGCCAGTGCCCCGAAGGATTTGCTGGG
     301 ---------+---------+---------+---------+---------+---------+ 360
         ACGGTCGTCCGGGACATGAAGAGTCTAAAGCACACGGTCACGGGGCTTCCTAAACGACCC

     aa    C  Q  Q  A  L  Y  F  S  D  F  V  C  Q  C  P  E  G  F  A  G  -


         AAGTGCTGTGAAATAGATACCAGGGCCACGTGCTACGAGGACCAGGGCATCAGCTACAGG
     361 ---------+---------+---------+---------+---------+---------+ 420
         TTCACGACACTTTATCTATGGTCCCGGTGCACGATGCTCCTGGTCCCGTAGTCGATGTCC

     aa    K  C  C  E  I  D  T  R  A  T  C  Y  E  D  Q  G  I  S  Y  R  -


         GGCACGTGGAGCACAGCGGAGAGTGGCGCCGAGTGCACCAACTGGAACAGCAGCGCGTTG
     421 ---------+---------+---------+---------+---------+---------+ 480
         CCGTGCACCTCGTGTCGCCTCTCACCGCGGCTCACGTGGTTGACCTTGTCGTCGCGCAAC

     aa    G  T  W  S  T  A  E  S  G  A  E  C  T  N  W  N  S  S  A  L  -


         GCCCAGAAGCCCTACAGCGGGCGGAGGCCAGACGCCATCAGGCTGGGCCTGGGGAACCAC
     481 ---------+---------+---------+---------+---------+---------+ 540
         CGGGTCTTCGGGATGTCGCCCGCCTCCGGTCTGCGGTAGTCCGACCCGGACCCCTTGGTG

     aa    A  Q  K  P  Y  S  G  R  R  P  D  A  I  R  L  G  L  G  N  H  -
```

```
          AACTACTGCAGAAACCCAGATCGAGACTCAAAGCCCTGGTGCTACGTCTTTAAGGCGGGG
     541  ---------+---------+---------+---------+---------+---------+ 600
          TTGATGACGTCTTTGGGTCTAGCTCTGAGTTTCGGGACCACGATGCAGAAATTCCGCCCC

     aa     N  Y  C  R  N  P  D  R  D  S  K  P  W  C  Y  V  F  K  A  G  -
```

```
          AAGTACAGCTCAGAGTTCTGCAGCACCCCTGCCTGCTCTGAGGGAAACAGTGACTGCTAC
     601  ---------+---------+---------+---------+---------+---------+ 660
          TTCATGTCGAGTCTCAAGACGTCGTGGGGACGGACGAGACTCCCTTTGTCACTGACGATG

     aa     K  Y  S  S  E  F  C  S  T  P  A  C  S  E  G  N  S  D  C  Y  -
```

```
          TTTGGGAATGGGTCAGCCTACCGTGGCACGCACAGCCTCACCGAGTCGGGTGCCTCCTGC
     661  ---------+---------+---------+---------+---------+---------+ 720
          AAACCCTTACCCAGTCGGATGGCACCGTGCGTGTCGGAGTGGCTCAGCCCACGGAGGACG

     aa     F  G  N  G  S  A  Y  R  G  T  H  S  L  T  E  S  G  A  S  C  -
```

```
          CTCCCGTGGAATTCCATGATCCTGATAGGCAAGGTTTACACAGCACAGAACCCCAGTGCC
     721  ---------+---------+---------+---------+---------+---------+ 780
          GAGGGCACCTTAAGGTACTAGGACTATCCGTTCCAAATGTGTCGTGTCTTGGGGTCACGG

     aa     L  P  W  N  S  M  I  L  I  G  K  V  Y  T  A  Q  N  P  S  A  -
```

```
          CAGGCACTGGGCCTGGGCAAACATAATTACTGCCGGAATCCTGATGGGGATGCCAAGCCC
     781  ---------+---------+---------+---------+---------+---------+ 840
          GTCCGTGACCCGGACCCGTTTGTATTAATGACGGCCTTAGGACTACCCCTACGGTTCGGG

     aa     Q  A  L  G  L  G  K  H  N  Y  C  R  N  P  D  G  D  A  K  P  -
```

```
     TGGTGCCACGTGCTGAAGAACCGCAGGCTGACGTGGGAGTACTGTGATGTGCCCTCCTGC
841  ---------+---------+---------+---------+---------+---------+ 900
     ACCACGGTGCACGACTTCTTGGCGTCCGACTGCACCCTCATGACACTACACGGGAGGACG

aa      W   C   H   V   L   K   N   R   R   L   T   W   E   Y   C   D   V   P   S   C   -


     TCCACCTGCGGCCTGAGACAGTACAGCCAGCCTCAGTTTCGCATCAAAGGAGGGCTCTTC
901  ---------+---------+---------+---------+---------+---------+ 960
     AGGTGGACGCCGGACTCTGTCATGTCGGTCGGAGTCAAAGCGTAGTTTCCTCCCGAGAAG

aa      S   T   C   G   L   R   Q   Y   S   Q   P   Q   F   R   I   K   G   G   L   F   -


     GCCGACATCGCCTCCCACCCCTGGCAGGCTGCCATCTTTGCCAAGCACAGGAGGTCGCCC
961  ---------+---------+---------+---------+---------+---------+ 1020
     CGGCTGTAGCGGAGGGTGGGGACCGTCCGACGGTAGAAACGGTTCGTGTCCTCCAGCGGG

aa      A   D   I   A   S   H   P   W   Q   A   A   I   F   A   K   H   R   R   S   P   -


     GGAGAGCGGTTCCTGTGCGGGGGGCATACTCATCAGCTCCTGCTGGATTCTCTCTGCCGCC
1021 ---------+---------+---------+---------+---------+---------+ 1080
     CCTCTCGCCAAGGACACGCCCCCGTATGAGTAGTCGAGGACGACCTAAGAGAGACGGCGG

aa      G   E   R   F   L   C   G   G   I   L   I   S   S   C   W   I   L   S   A   A   -


     CACTGCTTCCAGGAGAGGTTTCCGCCCCACCACCTGACGGTGATCTTGGGCAGAACATAC
1081 ---------+---------+---------+---------+---------+---------+ 1140
     GTGACGAAGGTCCTCTCCAAAGGCGGGGTGGTGGACTGCCACTAGAACCCGTCTTGTATG

aa      H   C   F   Q   E   R   F   P   P   H   H   L   T   V   I   L   G   R   T   Y   -
```

16

```
      CGGGTGGTCCCTGGCGAGGAGGAGCAGAAATTTGAAGTCGAAAAATACATTGTCCATAAG
1141  ---------+---------+---------+---------+---------+---------+  1200
      GCCCACCAGGGACCGCTCCTCCTCGTCTTTAAACTTCAGCTTTTTATGTAACAGGTATTC

aa       R  V  V  P  G  E  E  E  Q  K  F  E  V  E  K  Y  I  V  H  K   -


      GAATTCGATGATGACACTTACGACAATGACATTGCGCTGCTGCAGCTGAAATCGGATTCG
1201  ---------+---------+---------+---------+---------+---------+  1260
      CTTAAGCTACTACTGTGAATGCTGTTACTGTAACGCGACGACGTCGACTTTAGCCTAAGC

aa       E  F  D  D  D  T  Y  D  N  D  I  A  L  L  Q  L  K  S  D  S   -


      TCCCGCTGTGCCCAGGAGAGCAGCGTGGTCCGCACTGTGTGCCTTCCCCCGGCGGACCTG
1261  ---------+---------+---------+---------+---------+---------+  1320
      AGGGCGACACGGGTCCTCTCGTCGCACCAGGCGTGACACACGGAAGGGGGCCGCCTGGAC

aa       S  R  C  A  Q  E  S  S  V  V  R  T  V  C  L  P  P  A  D  L   -


      CAGCTGCCGGACTGGACGGAGTGTGAGCTCTCCGGCTACGGCAAGCATGAGGCCTTGTCT
1321  ---------+---------+---------+---------+---------+---------+  1380
      GTCGACGGCCTGACCTGCCTCACACTCGAGAGGCCGATGCCGTTCGTACTCCGGAACAGA

aa       Q  L  P  D  W  T  E  C  E  L  S  G  Y  G  K  H  E  A  L  S   -


      CCTTTCTATTCGGAGCGGCTGAAGGAGGCTCATGTCAGACTGTACCCATCCAGCCGCTGC
1381  ---------+---------+---------+---------+---------+---------+  1440
      GGAAAGATAAGCCTCGCCGACTTCCTCCGAGTACAGTCTGACATGGGTAGGTCGGCGACG

aa       P  F  Y  S  E  R  L  K  E  A  H  V  R  L  Y  P  S  S  R  C   -
```

```
      ACATCACAACATTTACTTAACAGAACAGTCACCGACAACATGCTGTGTGCTGGAGACACT
1441  ---------+---------+---------+---------+---------+---------+  1500
      TGTAGTGTTGTAAATGAATTGTCTTGTCAGTGGCTGTTGTACGACACACGACCTCTGTGA

 aa     T   S   Q   H   L   L   N   R   T   V   T   D   N   M   L   C   A   G   D   T   -
                                                                                        .


      CGGAGCGGCGGGCCCCAGGCAAACTTGCACGACGCCTGCCAGGGCGATTCGGGAGGCCCC
1501  ---------+---------+---------+---------+---------+---------+  1560
      GCCTCGCCGCCCGGGGTCCGTTTGAACGTGCTGCGGACGGTCCCGCTAAGCCCTCCGGGG

 aa     R   S   G   G   P   Q   A   N   L   H   D   A   C   Q   G   D   S   G   G   P   -


      CTGGTGTGTCTGAACGATGGCCGCATGACTTTGGTGGGCATCATCAGCTGGGGCCTGGGC
1561  ---------+---------+---------+---------+---------+---------+  1620
      GACCACACAGACTTGCTACCGGCGTACTGAAACCACCCGTAGTAGTCGACCCCGGACCCG

 aa     L   V   C   L   N   D   G   R   M   T   L   V   G   I   I   S   W   G   L   G   -


      TGTGGACAGAAGGATGTCCCGGGTGTGTACACCAAGGTTACCAACTACCTAGACTGGATT
1621  ---------+---------+---------+---------+---------+---------+  1680
      ACACCTGTCTTCCTACAGGGCCCACACATGTGGTTCCAATGGTTGATGGATCTGACCTAA

 aa     C   G   Q   K   D   V   P   G   V   Y   T   K   V   T   N   Y   L   D   W   I   -
                                      .


      CGTGACAACATGCGA
1681  ---------+-----
      GCACTGTTGTACGCT

 aa   R   D   N   M   R   P   *
```

18

TABLE 3

| DESIGNATION OF TPA DOMAINS | | |
|---|---|---|
| (after Degan et al . 1986) | | |
| | Nucleotide | Amino Acid |
| Propeptide/signal | 22 to 108 | - 29 to - 1 |
| Finger domain | 133 to 246 | 9 to 46 |
| Growth factor domain | 268 to 367 | 54 to 87 |
| Kringle 1 domain | 391 to 634 | 95 to 176 |
| Kringle 2 domain | 655 to 900 | 183 to 264 |
| Protease domain | 943 to 1698 | 279 to 530 |

## TABLE 4

### mtPAs

L309V (with primer PP1)

|     | 306 | 307 | 308 | 309 | 310 | 311 | 312 |
|-----|-----|-----|-----|-----|-----|-----|-----|
|     | Glu | Arg | Phe | Val | Cys | Gly | Gly |
|     | GAG | CGG | TTC | GTT | TGC | GGG | GGC |
|     | 1024 |    |     |     |     |     | 1044 |

E329D (with primer PP2)

|     | 326 |     |     |     |     |     | 332 |
|-----|-----|-----|-----|-----|-----|-----|-----|
|     | Cys | Phe | Gln | Asp | Arg | Phe | Pro |
|     | TGC | TTC | CAG | GAT | CGG | TTT | CCG |
|     | 1084 |    |     |     |     |     | 1104 |

D368A (with primer PP3)

|     | 364 |     |     |     |     |     | 370 |
|-----|-----|-----|-----|-----|-----|-----|-----|
|     | Lys | Glu | Phe | Asp | Ala | Asp | Thr |
|     | AAG | GAG | TTC | GAT | GCC | GAC | ACT |
|     | 1198 |    |     |     |     |     | 1218 |

Y371L (with primer PP4)

```
        368                                    374
        Asp  Asp  Thr  Leu  Asp  Asn  Asp


        GAT  GAC  ACG  CTC  GAC  AAT  GAC
        1210                               1230
```

V436E (with primer PP5E)

```
        433                                    439
        Glu  Ala  His  Glu  Arg  Leu  Tyr


        GAG  GCT  CAT  GAG  AGA  CTG  TAC
        1405                               1425
```

V436Q (with primer PP5Q)

```
        433                                    39
        Glu  Ala  His  Gln  Arg  Leu  Tyr


        GAG  GCT  CAT  CAG  AGA  CTG  TAC
        1405                               1425
```

G462S (with primer PP6S)

```
        459                                    465
        Leu  Cys  Ala  Ser  Asp  Thr  Arg
```

```
      CTG  TGT  GCT  AGC  GAC  ACT  CGG
      1483                     1503
```

G462T (with primer PP6T)

```
      459                     465
      Leu  Cys  Ala  Thr  Asp  Thr  Arg


      CTG  TGT  GCT  ACA  GAC  ACT  CGG
      1483                     1503
```

G462D (with primer PP6D)

```
      459                     465
      Leu  Cys  Ala  Asp  Asp  Thr  Arg
                     -

      CTG  TGT  GCT  GAC  GAC  ACT  CGG
      1483                     1503
```

RS465,466LP (with primer PP7)

```
      462                                     470
      Gly  Asp  Thr  Leu  Pro  Gly  Gly  Pro  Gln


      GGA  GAC  ACT  CTG  CCC  GGC  GGC  CCC  CAG
      1492                                         1518
```

## A476S (with primer PP8)

```
        473                              479
        Leu  His  Asp  Ser  Cys  Gln  Gly

        TTG  CAC  GAC  AGC  TGC  CAG  GGC
        1525                             1545
```

## I499V (with primer PP9)

```
        496                              502
        Val  Gly  Ile  Val  Ser  Trp  Gly

        GTG  GGC  ATC  GTG  AGC  TGG  GGC
        1594                             1604
```

## G506A (with primer PP10A)

```
        503                              509
        Leu  Gly  Cys  Ala  Gln  Lys  Asp

        CTG  GGC  TGT  GCT  CAG  AAG  GAT
        1615                             1635
```

## G506S (with primer PP10S)

```
        503                              509
        Leu  Gly  Cys  Ser  Gln  Lys  Asp

        CTG  GGC  TGT  AGT  CAG  AAG  GAT
        1615                             1635
```

<u>P511D</u> (with primer PP11D)

```
        508                        514
        Lys  Asp  Val  Asp  Gly  Val  Tyr

        AAG  GAT  GTC  GAC  GGT  GTG  TAC
        1630                       1650
```

<u>P511N</u> (with primer PP11N)

```
        508                        514
        Lys  Asp  Val  Asn  Gly  Val  Tyr

        AAG  GAT  GTG  CAG  GGT  GTG  TAC
        1630                       1650
```

## Claims

1. A recombinant polypeptide having natural tissue plasminogen activator fibrinolytic-like activity wherein the nucleic acid sequence encoding the recombinant polypeptide comprises a nucleic acid sequence modified from the naturally occurring tPA sequence in the protease domain.

2. A polypeptide having natural tissue plasminogen activator fibrinolytic-like activity and one or more modifications in the protease domain.

3. A polypeptide as claimed in claim 1 or 2 wherein the modification results in a sequence more homologous to that of the naturally occurring nucleic acid sequence encoding the protease domain of urokinase plasminogen activator.

4. A polypeptide as claimed in any of claims 1 to 3 comprising a polypeptide encoded by the sequence L309V, E329D, D368A, Y371L, V436E, V436Q, G462S, G462T, G462D, A476S, I499V, G506A, G506S, P511D, P511N or RS465,466LP.

5. A nucleic acid sequence encoding for a polypeptide as claimed in any of claims 1 to 4.

6. A nucleic acid sequence for encoding a polypeptide having tPA fibrinolytic-like activity comprising a modified protease domain, said sequence comprising the sequence:
GAG CGG TTC GTT TGC GGG GGC,
TGC TTC CAG GAT CGG TTT CCG,
AAG GAG TTC GAT GCC GAC ACT,
GAT GAC ACG CTC GAC AAT GAC,
GAG GCT CAT GAG AGA CTG TAC,
GAG GCT CAT CAG AGA CTG TAC,
CTG TGT GCT AGC GAC ACT CGG,
CTG TGT GCT ACA GAC ACT CGG,
CTG TGT GCT GAC GAC ACT CGG,
GGA GAC ACT CTG CCC GGC GGC CCC CAG,
TTG CAC GAC AGC TGC CAG GGC,
GTG GGC ATC GTG AGC TGG GGC,
CTG GGC TGT GCT CAG AAG GAT,
CTG GGC TGT AGT CAG AAG GAT,
AAG GAT GTC GAC GGT GTG TAC, or
AAG GAT GTG CAG GGT GTG TAC.

7. A sequence as claimed in claim 6 further comprising a codon substituted in place of at least one of the codons in a DNA sequence encoding an ASN-X-(Ser or Thr) signal for N-linked glycosylation wherein the substituted codon encodes an animo acid, the substitution of which prevents glycosylation at such signal.

8. An expression vector comprising a nucleic acid sequence as claimed in any of claims 5 to 7.

9. A host transfected with a vector as claimed in claim 8.

10. A polypeptide as claimed in any of claims 1 to 4 for use in medicine.

11. A pharmaceutical composition comprising a polypeptide as claimed in any of claims 1 to 4 and a pharmaceutically acceptable carrier.

Claims for the following Contracting State : ES

1. A process for the preparation of a recombinant polypeptide having natural tissue plasminogen activator fibrinolytic-like activity comprising modifying the nucleic acid sequence encoding the recombinant polypeptide from the naturally occurring tPA sequence in the protease domain.

2. A process for the preparation of a modified polypeptide having natural tissue plasminogen activator fibrinolytic-like activity, comprising making one or more modifications in the protease domain.

3 A process as claimed in claim 1 or 2 wherein the modification results in a sequence more homologous to that of the naturally occurring nucleic acid sequence encoding the protease domain of urokinase plasminogen activator.

4. A process as claimed in any of claims 1 to 3 wherein the recombinant polypeptide comprises a polypeptide encoded by the sequence L309V, E329D, D368A, Y371L, V436E, V436Q, G462S, G462T, G462D, A476S, I499V, G506A, G506S, P511D, P511N or RS465,466LP.

5. A process for the preparation of a nucleic acid sequence encoding for a polypeptide as prepared in any of claims 1 to 4, the process comprising coupling successive nucleotides together.

6. A process for the preparation of a nucleic acid sequence for encoding a polypeptide having tPA fibrinolytic-like activity having a modified protease domain, the sequence comprising the sequence:

GAG CGG TTC GTT TGC GGG GGC,
TGC TTC CAG GAT CGG TTT CCG,
AAG GAG TTC GAT GCC GAC ACT,
GAT GAC ACG CTC GAC AAT GAC,
GAG GCT CAT GAG AGA CTG TAC,
GAG GCT CAT CAG AGA CTG TAC,
CTG TGT GCT AGC GAC ACT CGG,
CTG TGT GCT ACA GAC ACT CGG,
CTG TGT GCT GAC GAC ACT CGG,
GGA GAC ACT CTG CCC GGC GGC CCC CAG,
TTG CAC GAC AGC TGC CAG GGC,
GTG GGC ATC GTG AGC TGG GGC,
CTG GGC TGT GCT CAG AAG GAT,
CTG GGC TGT AGT CAG AAG GAT,
AAG GAT GTC GAC GGT GTG TAC, or
AAG GAT GTG CAG GGT GTG TAC;
the process comprising coupling successive nucleotides together.

7. A process as claimed in claim 6 wherein the sequence further comprises a codon substituted in place of at least one of the codons in a DNA sequence encoding an ASN-X-(Ser or Thr) signal for N-linked glycosylation wherein the substituted codon encodes an animo acid, the substitution of which prevents glycosylation at such signal.

8. A process for the preparation of an expression vector comprising a nucleic acid sequence as prepared in any of claims 5 to 7, the process comprising coupling successive nucleotides together.

9. A process for the preparation of a host comprising transfecting a host with a vector prepared in claim 8.

10. The use of a polypeptide as prepared in any of claims 1 to 4 in the preparation of a fibrinolytic agent.

11. A process for the preparation of a pharmaceutical composition comprising admixing a polypeptide as prepared in any of claims 1 to 4 with a pharmaceutically acceptable carrier.

Claims for the following Contracting State : GR

25

1. A process for the preparation of a recombinant polypeptide having natural tissue plasminogen activator fibrinolytic-like activity comprising modifying the nucleic acid sequence encoding the recombinant polypeptide from the naturally occurring tPA sequence in the protease domain.

2. A process for the preparation of a modified polypeptide having natural tissue plasminogen activator fibrinolytic-like activity, comprising making one or more modifications in the protease domain.

3. A process as claimed in claim 1 or 2 wherein the modification results in a sequence more homologous to that of the naturally occurring nucleic acid sequence encoding the protease domain of urokinase plasminogen activator.

4. A process as claimed in any of claims 1 to 3 wherein the recombinant polypeptide comprises a polypeptide encoded by the sequence L309V, E329D, D368A, Y371L, V436E, V436Q, G462S, G462T, G462D, A476S, I499V, G506A, G506S, P511D, P511N or RS465,466LP.

5. A nucleic acid sequence encoding for a polypeptide as claimed in any of claims 1 to 4.

6. A nucleic acid sequence for encoding a polypeptide having tPA fibrinolytic-like activity comprising a modified protease domain, said sequence comprising the sequence:

GAG CGG TTC GTT TGC GGG GGC,
TGC TTC CAG GAT CGG TTT CCG,
AAG GAG TTC GAT GCC GAC ACT,
GAT GAC ACG CTC GAC AAT GAC,
GAG GCT CAT GAG AGA CTG TAC,
GAG GCT CAT CAG AGA CTG TAC,
CTG TGT GCT AGC GAC ACT CGG,
CTG TGT GCT ACA GAC ACT CGG,
CTG TGT GCT GAC GAC ACT CGG,
GGA GAC ACT CTG CCC GGC GGC CCC CAG,
TTG CAC GAC AGC TGC CAG GGC,
GTG GGC ATC GTG AGC TGG GGC,
CTG GGC TGT GCT CAG AAG GAT,
CTG GGC TGT AGT CAG AAG GAT,
AAG GAT GTC GAC GGT GTG TAC, or
AAG GAT GTG CAG GGT GTG TAC.

7. A sequence as claimed in claim 6 further comprising a codon substituted in place of at least one of the codons in a DNA sequence encoding an ASN-X-(Ser or Thr) signal for N-linked glycosylation wherein the substituted codon encodes an animo acid, the substitution of which prevents glycosylation at such signal.

8. An expression vector comprising a nucleic acid sequence as claimed in any of claims 5 to 7.

9. A host transfected with a vector as claimed in claim 8.

10. The use of a polypeptide as prepared in any of claims 1 to 4 in the preparation of a fibrinolytic agent.

11. A process for the preparation of a pharmaceutical composition comprising admixing a polypeptide as prepared in any of claims 1 to 4 with a pharmaceutically acceptable carrier.

Fig 1

# Fig 2

# Fig 3

MT-1 Promoter    13286 Xhol    SV40 E PolyA

12586 Kpnl    Hpal 13421    BamHI 13529/1

11286 EcoRI

11260 HindIII    Xbal 1682

10286 EcoRI    CLH3AXBPV    HindIII 2508

Hpal 3495

Smal 4440

9658 Pvul

pML-1    BPV-1

7945 Sall    EcoRI 5608

Kpnl 6950